Europäisches Patentamt

(19) European Patent Office

Office européen des brevets

(11) Numéro de publication: **0 091 846**
**B1**

(12) **FASCICULE DE BREVET EUROPEEN**

(45) Date de publication du fascicule du brevet:
**29.01.86**

(21) Numéro de dépôt: **83400561.3**

(22) Date de dépôt: **17.03.83**

(51) Int. Cl.⁴: **A 61 B 17/34**

(54) **Dispositif pour le repérage de l'espace péridural.**

(30) Priorité: **08.04.82 FR 8206169**

(43) Date de publication de la demande:
**19.10.83 Bulletin 83/42**

(45) Mention de la délivrance du brevet:
**29.01.86 Bulletin 86/5**

(84) Etats contractants désignés:
**BE DE GB NL**

(56) Documents cités:
**FR - A - 715 653**
**FR - A - 1 544 767**
**FR - A - 2 365 350**
**US - A - 3 081 770**

(73) Titulaire: **Société VYGON, 5-11 rue Adeline,**
**F-95440 Ecouen (FR)**

(72) Inventeur: **Persat, Jean-Charles, 4, allée Maurice Ravel,**
**F-95230 Soisy Sous Montmorency (FR)**

(74) Mandataire: **Martin, Jean-Jacques et al, Cabinet**
**REGIMBEAU 26, Avenue Kléber, F-75116 Paris (FR)**

# Description

La présente invention concerne un dispositif pour le repérage de l'espace péridual.

Le repérage de l'espace péridual, effectué pour une anesthésie péridurale, constitue une opération particulièrement délicate.

En effet, l'anesthésie péridurale est réalisée par injection d'un anesthésique local dans l'espace péridual et le succès de cette opération dépend donc de la possibilité d'atteindre cet espace anatomique par une ponction percutanée.

Le dispositif qui fait l'objet de la présente invention permet précisément de repérer de façon simple tout en minimisant les risques de complication, l'instant où l'aiguille de ponction pénètre dans l'espace péridual.

Afin de bien comprendre le fonctionnement du dispositif de l'invention, il est nécessaire de décrire brièvement l'espace péridual.

L'espace péridual est un espace étroit de quelques millimètres d'épaisseur, constitué de tissus graisseux et d'un réseau vasculaire artériel, veineux et lymphatique. Ce tissu constitue un milieu peu dense que l'on peut distendre sous l'action d'une faible tension. Il y règne une pression relativement faible, nulle ou négative.

L'espace péridual est habituellement ponctionné dans son segment postérieur. A ce niveau, il est limité en avant par la dure-mère, en arrière par un tissu ligamentaire dense qui ne peut être distendu qu'en utilisant de fortes tensions.

Le dispositif de l'invention permet de repérer facilement l'espace péridual en tirant parti de la pression relativement faible qui règne dans cet espace.

Selon la caractéristique essentielle de l'invention, le dispositif comprend un raccord à trois branches comprenant une branche distale apte à recevoir une aiguille de ponction, une branche proximale principale pourvue d'un ballonnet, et une branche proximale latérale pourvue d'une valve anti-retour dont l'entrée est apte à être raccordée à une seringue.

Ainsi, lorsque l'aiguille de ponction est piquée dans les plans cutanés de l'espace à repérer, l'actionnement du piston de la seringue provoque le gonflement du ballonnet qui reste gonflé, après retrait de la seringue, du fait de la valve anti-retour. Dès que le biseau de l'aiguille atteint l'espace péridual, le ballonnet se dégonfle en visualisant ainsi l'instant précis où l'espace péridual est repéré par l'aiguille.

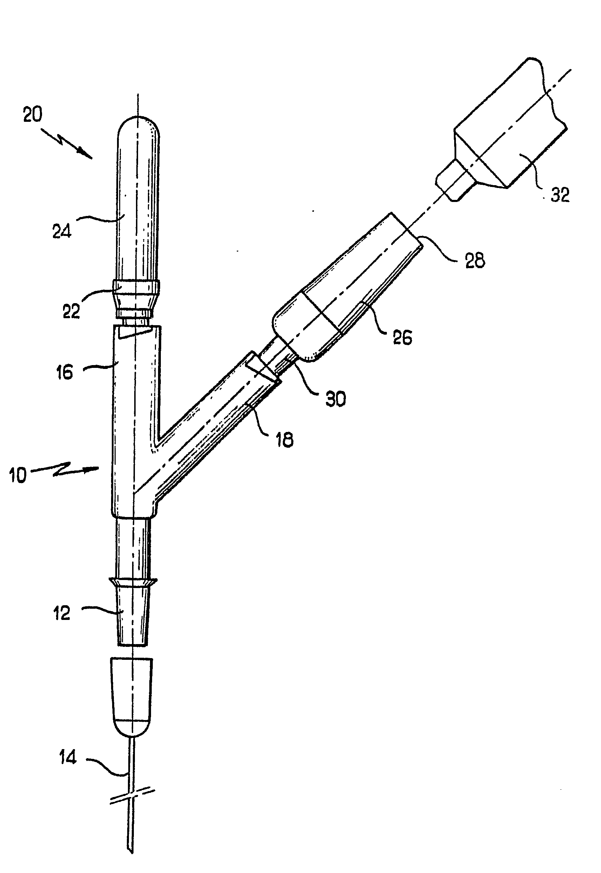

D'autres caractéristiques et avantages de l'invention seront mieux compris à la lecture de la description détaillée qui suit et en référence au dessin annexé, donné uniquement à titre illustratif de l'invention, sur lequel:

La figure unique est une vue en élévation d'un dispositif de repérage selon l'invention.

Le dispositif représenté sur le dessin annexé comprend un raccord 10 à dérivation latérale. Il s'agit d'un raccord à trois branches comprenant une branche distale 12 destinée à recevoir une aiguille de ponction 14, une branche proximale principale 16 et une branche proximale latérale 18.

La branche proximale principale 16 est pourvue d'un ballonnet 20 constitué d'un embout rigide 22 sur lequel est fixé, par exemple au moyen de ligatures, le corps du ballonnet ou ballonnet proprement dit 24. Ce dernier est constitué d'une matière souple, élastique et imperméable, par exemple de latex.

La branche proximale latérale 18 est munie d'une valve anti-retour 26, par exemple du type à membrane, qui laisse passer les fluides depuis son entrée proximale 28 vers sa sortie distale 30, qui est elle-même emmanchée sur la branche 18. La valve anti-retour 26 s'oppose à tout passage de fluide en sens opposé.

L'entrée 28 de la valve 26 est prévue pour être raccordée à une seringue classique 32.

Le dispositif de l'invention s'utilise de la façon suivante: on monte l'aiguille de ponction 14 sur la branche distale 12 du raccord 10. On branche ensuite dans l'entrée 28 de la valve anti-retour 26 la seringue 32 dont le piston a été retiré de façon à définir un volume équivalent au volume de gonflage du ballonnet 20.

L'aiguille de ponction 14 est alors piquée dans les plans cutanés. On enfonce ensuite le piston de la seringue de façon à gonfler le ballonnet 20, puis on retire la seringue de la valve anti-retour 26.

La traversée des plans cutanés, sous-cutanés musculaires et ligamentaires se fait sans variation du volume du ballonnet, car les tissus rencontrés s'opposent à son dégonflement.

Dès que le biseau de l'aiguille 14 atteint l'espace péridual, le ballonnet peut y évacuer une partie de son contenu, car la pression interne dans le ballonnet est à ce moment supérieure à celle de l'espace péridual.

Le dégonflement du ballonnet permet de visualiser l'instant où l'aiguille atteint l'espace péridual.

On peut alors procéder à l'injection d'un anesthésique au moyen de l'aiguille de ponction qui se trouve correctement implantée dans l'espace péridual.

Les caractéristiques du ballonnet sont étudiées de manière à n'introduire dans l'espace péridual qu'un très faible volume d'air sous une faible pression.

# Revendications

1. Dispositif pour le repérage de l'espace péridual, caractérisé par le fait qu'il comprend un raccord (10) à trois branches comprenant une branche distale (12) apte à recevoir une aiguille de ponction (14), une branche proximale principale (16) pourvue d'un ballonnet (20), et une branche proximale latérale (18) pourvue d'une valve anti-retour (26) dont l'entrée (28) est apte à être raccordée à une seringue (32), en sorte que l'actionnement du piston de la seringue provoque le gonflement du ballonnet lorsque l'aiguille de ponction est piquée dans les plans cutanés de l'espace à repérer, la pénétration de l'aiguille dans l'espace péridual étant alors visualisée par le dégonflement du ballonnet.

2. Dispositif selon la revendication 1, caractérisé par le fait que le ballonnet (20) comprend un embout rigide (22) sur lequel est monté le corps (24) du ballonnet.

**Patentansprüche**

1. Einrichtung zum Lokalisieren des Periduralraumes, dadurch gekennzeichnet, dass sie ein Verbindungsstück (10) mit drei Zweigen aufweist, welches einen distalen Zweig (12) zur Aufnahme einer Punktionsnadel (14), einen mit einem kleinen Ballon (20) versehenen proximalen Hauptzweig (16), und einen proximalen Seitenzweig (18) aufweist, der mit einem Anti-Rückstrom-Ventil (26) versehen ist, dessen Eingang (28) mit einer Spritze (32) verbunden werden kann, so dass die Betätigung des Kolbens der Spritze die Füllung des kleinen Ballons bewirkt, wenn die Punktionsnadel in die Hautebene des zu lokalisierenden Raumes gestochen wird, wobei das Eindringen der Nadel in den Periduralraum dann durch die Entleerung des kleinen Ballons sichtbar wird.

2. Einrichtung nach Anspruch 1, dadurch gekennzeichnet, dass der kleine Ballon (20) ein starres Übergangsrohrstück (22) aufweist, auf welchem der Körper (24) des kleinen Ballons montiert ist.

**Claims**

1. Device for locating the peridural space, characterised by the fact that it comprises a coupling (10) with three branches comprising a distal branch (12) able to receive a puncture needle (14), a main proximal branch (16) provided with an inflatable bag (20) and a lateral proximal branch (18) provided with a non-return valve (26) whereof the inlet (28) is able to be connected to a syringe (32), in order that the actuation of the piston of the syringe causes the inflation of the inflatable bag when the puncture needle is inserted in the cutaneous surfaces of the space to be located, the penetration of the needle into the peridural space thus being visualised by the deflation of the inflatable bag.

2. Device according to Claim 1, characterised by the fact that the inflatable bag (20) comprises a rigid mouth-piece (22) to which the body (24) of the inflatable bag is fitted.